# EUROPÄISCHE PATENTSCHRIFT

(11) **EP 0 407 737 B1**
(45) Veröffentlichungstag und Bekanntmachung des Hinweises auf die Patenterteilung: **11.08.1993**
(21) Anmeldenummer: 90110748.2
(22) Anmeldetag: 07.06.1990
(51) Int. Cl.: A61M 1/16

(54) **Verfahren zur Prüfung einer Hämodialysemembran**
Method for testing a hemodialysis membrane
Procédé pour examiner une membrane d'hémodialyse

(30) Priorität: 13.07.1989 DE 3923078
(43) Veröffentlichungstag der Anmeldung: 16.01.1991
(73) Patentinhaber: Fresenius AG, 61350 Bad Homburg (DE)
(72) Erfinder: Polascheeg, Hans-Dietrich, Dr., D-6370 Oberursel 4 (DE)
(74) Vertreter: Fuchs Mehler Weiss

(56) Entgegenhaltungen:
- DE-A- 3 442 744

## Beschreibung

Die Erfindung bezieht sich auf ein Verfahren zur Prüfung einer Hämodialysemembran eines Dialysators, welcher eine Blutkammer und eine Dialysierflüssigkeitskammer aufweist, bei welchem die in der Blutkammer befindliche Flüssigkeit durch Luft ersetzt wird und in einem ersten Prüfschritt die Blutseite mit einem Überdruck beaufschlagt wird.

Die Hämodialyse stellt ein Verfahren dar, welches seit über 25 Jahren erfolgreich zur Behandlung einer großen Anzahl von Patienten weltweit angewendet wird. Bedingt durch die hohe Anzahl von Patienten, welche mittels der Hämodialyse behandelt werden, ist es notwendig, die benötigten Geräte oder Vorrichtungen in großer Anzahl bereit zu stellen. Die technische Versorgung stellt in den Industriestaaten üblicherweise kein Problem dar, die Länder der Dritten Welt oder die Schwellenländer sind jedoch besonders von Versorgungsengpässen betroffen. Da sich die Zuverlässigkeit und der Ausnutzungsgrad der Dialyseeinrichtungen nicht nur hinsichtlich der technischen Qualität der Dialyse und der erforderlichen Zeit auswirkt, sondern auch hinsichtlich der volkswirtschaftlichen Belastung, sind erhebliche Anstrengungen erforderlich, um die technische Verfügbarkeit und den Aufwand, welcher zur Dialysebehandlung zu betreiben ist, zu senken. Ein dabei zusätzlich auftretender Effekt liegt in der Senkung der entstehenden Kosten.

Um die technische Verfügbarkeit von Dialyseeinrichtungen zu erhöhen und diese mit einem hohen Wirkungsgrad zu betreiben, wurde versucht, die Dialysatoren mehrfach zu verwenden, das heißt nach Benutzung durch einen Patienten entsprechend zu reinigen und bei einem nachfolgenden Patienten einzusetzen. Der Vorteil dieser Vorgehensweise liegt darin, daß der Dialysator solange verwendet werden kann, bis er funktionsunfähig wird oder aus Sicherheitsgründen ausgetauscht werden muß. Ein Austausch oder Ersatz eines nur zum Teil verbrauchten Dialysators kann somit vermieden werden.

Um einen Dialysator wieder verwenden zu können, ist es unumgänglich, dessen Funktionsfähigkeit vor der erneuten Verwendung zu überprüfen. Dabei ist es insbesondere erforderlich, die Membran hinsichtlich ihrer Intaktheit einer Überprüfung zu unterwerfen. Eine derartige Prüfung erfolgt üblicherweise unmittelbar nach einer vorangegangenen Hämodialysebehandlaung und nach der Reinigung des Dialysators, bevor dieser mit einer desinfizierenden Lösung, beispielsweise Formaldehyd, gefüllt wird.

Aus der DE-PS 3442744 ist ein Verfahren zum Ermitteln von Lecks in der Membran bekannt, bei welchem die Blutseite oder Blutkammer des Dialysators bei feuchter Membran mit Luft beaufschlagt wird, welche einen Überdruck aufweist. Sollte die Membran defekt sein und Fehlstellen oder Löcher aufweisen, so können die Luftblasen von der Blutkammer in die Dialysierflüssigkeitskammer hindurchtreten. Sofern letztere mit Flüssigkeit gefüllt ist, können die Luftblasen erkannt werden. Es ist jedoch auch möglich, die blutseitig angeschlossene Apparatur nach der Druckbeaufschlagung der Blutkammer durch Luft abzudichten, um den Druck in der Blutkammer zu überwachen. Bei einem Leck der Membran tritt ein Druckabfall auf der Blutseite auf. Dieser Druckabfall kann über einen Sensor festgestellt und einer Vorrichtung zur automatischen Überprüfung des Dialysators zugeführt werden.

Alternativ dazu ist es auch möglich, in der mit Flüssigkeit gefüllten Dialysierflüssigkeitskammer einen Unterdruck zu erzeugen und die Blutkammer zu belüften. Auch hierbei können Fehlstellen oder Lacks in der Membran durch Veränderungen des Unterdrucks in der Dialysierflüssigkeitskammer ermittelt werden.

Bei den bekannten Prüfungsverfahren wird davon ausgegangen, daß die Fehlstelle oder das Leck der Membran unabhängig von der Druckbeaufschlagung ist, das heißt unabhängig davon bemerkt werden kann, in welche Richtung der Druckgradient verläuft. Weiterhin wird auch davon ausgegangen, daß die Fehlstelle oder das Leck der Membran bei einem Überdruck auf der Blutseite, das heißt der Blutkammer ermittelt werden kann. Diese Annahmen sind für die meisten Defekte von Membranen zutreffend, so daß die Prüfungverfahren üblicherweise erfolgreich zur Ermittlung von Defekten der Membran eingesetzt werden können.

Bei hochpermeablen Hämodialysemembranen tritt jedoch ein Effekt auf, welcher die Anwendung dieser Prüfverfahren nicht zuläßt. Es können nämlich bei der Hämodialyse bei derartigen hochpermeablen Membranen Bereiche im Dialysator existieren, bei denen ein umgekehrter Druckgradient vorherrscht, das heißt auf der Dialysierflüssigkeitsseite ein höherer Druck vorliegt, als auf der Blutseite. Als Folge davon kommt es zur sogenannten umgekehrten Ultrafiltration. Bei diesen Membranen ist nicht auszuschließen, daß Defekte der Membran existieren, welche bei einer umgekehrten Druckbeaufschlagung zu einem Leck führen. Durch diese Leckagestellen kann während der Dialyse unbemerkt Dialysierflüssigkeit in den Blutkreislauf eindringen. Sofern die Dialysierflüssigkeit kontaminiert ist, kann dieser Übertritt in die Blutseite zu pyrogenen Reaktionen beim Patienten oder sogar zur Sepsis führen.

Der Erfindung liegt die Aufgabe zugrunde, ein Verfahren zur Prüfung einer Hämodialysesmembran zu schaffen, welches bei einfachem Aufbau und betriebssicherer Anwendbarkeit eine vollständige Überprüfung der Membran ermöglicht.

Erfindunggemäß wird die Aufgabe dadurch gelöst, daß die Dialysierflüssigkeitskammer vor dem ersten Prüfschritt mit Luft gefüllt wird und daß in einem zweiten Prüfschritt in der Blutkammer ein Unterdruck erzeugt wird.

Bei dem erfindungsgemäßen Verfahren folgt somit die Prüfung der Membran in zwei Stufen, in der ersten Stufe wird in der Blutkammer ein Überdruck angelegt, um einen Druckgradienten von der Blutkammer zu der Dialysierflüssigkeitskammer zu erzeugen. In dem zweiten Prüfschritt wird auf die Membran ein entgegengesetzter Druckgradient aufgebracht. Erfindungsgemäß erfolgt somit eine doppelte Überprüfung der Membran, um Leckagestellen zu ermitteln, welche möglicherweise nur bei einem der beiden Druckgradienten auftreten oder bemerkt werden können.

In einer besonders günstigen Ausgestaltung des erfindungsgemäßen Verfahren ist vorgesehen, daß der Überdruck bzw. der Unterdruck in der Blutkammer etwa plus 500 mm/Hg bzw. minus 500 mm/Hg beträgt. Bei dieser Größenordnung ist zum einen sichergestellt, daß Leckagestellen in zuverlässiger Weise ermittelt werden können, zum anderen besteht bei diesem Druckgradienten nicht die Gefahr, daß Luft durch intakte Stellen der Membran gepreßt wird.

Bevorzugterweise erfolgt erfindungsgemäß eine Leckageanzeige, wenn der Überdruck und/oder der Unterdruck bei dem ersten und dem zweiten Prüfschritt nicht erreicht wurde. Es ist weiterhin besonders günstig, eine Leckageanzeige dann zu betätigen, wenn der Druckanstieg und/oder der Druckabfall etwa 20 mm/Hg beträgt.

Weiterhin kann es erfindungsgemäß besonders günstig sein, wenn vor Beginn der Prüfung die Membran mit Flüssigkeit gefüllt wird und vor dem zweiten Prüfschritt zur Verdrängung der in der Blutkammer befindlichen Flüssigkeit durch die Membran in die Dialysierflüssigkeitskammer die Blutkammer mit Luft gefüllt wird. Die Dialysierflüssigkeitskammer kann zum Ablauf der Flüssigkeit mit einer Ablaufleitung verbunden, daß heißt geöffnet werden.

Das erfindungsmäße Verfahren zeichnet sich durch hohes Maß an Einfachheit aus, so daß kein hoher beratungstechnischer Aufwand erforderlich ist, um das Prüfverfahren durchzuführen. Es ist somit möglich, die Prüfung unter geringem Aufwand automatisch auszuführen. Es ist erfindungsgemäß lediglich erforderlich, daß die Membran des Dialysators zunächst mit Flüssigkeit gefüllt, danach die Blut- und Dialysierflüssigkeitsseite mit Luft gefüllt, nachfolgend die Blutseite bei belüfteter Dialysierflüssigkeitsseite mit Luft bei einem Über- bzw. Unterdruck beaufschlagt wird und die Druckveränderung gemessen wird.

Im folgenden wird das erfindungsgemäße Verfahren anhand eines Ausführungsbeispiels einer erfindungsgemäßen Hämodialysevorrichtung in Verbindung mit der Zeichnung beschrieben. Dabei zeigt:

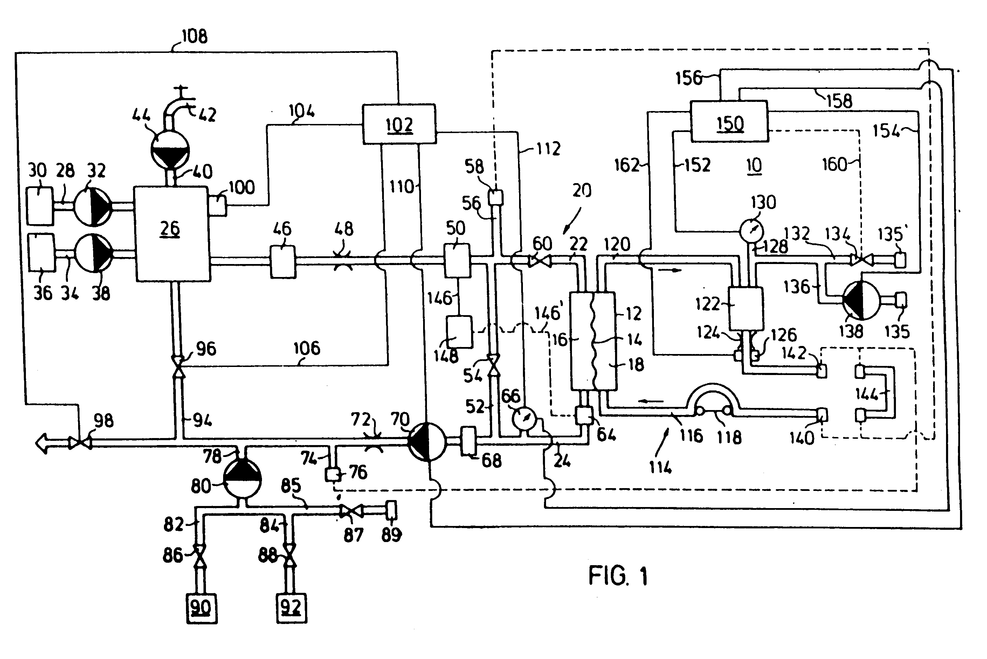

- Fig. 1: Eine schematische Darstellung einer Ausführungsform der erfindungsgemäßen Hämodialysevorrichtung mit einem Leckprüfgerät.

Die Fig. 1 ist ein Hämodialysegerät 10 in schematischer Weise dargestellt, welches einen Dialysator 12 umfasst, welcher mit einer Blutkammer 18 und einer Dialysierflüssigkeitskammer 16 versehen ist, welche durch eine Membran 14 voneinander getrennt sind. Die Dialysierflüssigkeitskammer wird von Dialysierflüssigkeit durchströmt, durch die Blutkammer 18 wird Patientenblut geleitet.

Die Kammer 16 ist in einem Dialysierflüssigkeitsweg 20 angeordnet, welcher aus einer Zuleitung 22, welche in die Kammer 16 des Dialysators 12 mündet, und einer Ableitung 24 besteht, welche auf der gegenüberliegenden Seite der Kammer 16 von dem Dialysator 12 abgeht.

Die Zuleitung 22 ist weiterhin mit einem Mischgefäß 26 verbunden, welches eine erste Zuleitung 28 aufweist, die mit einem ersten Konzentratbehälter 30 betriebsverbunden ist und in welche eine Pumpe 32 eingeschaltet ist. Weiterhin ist eine zweite Zuleitung 34 mit dem Mischgerät 26 verbunden, welche in einen zweiten Konzentratbehälter 36 mündet, wobei in der Zuleitung 34 eine weitere Pumpe 38 angeordnet ist. Die beiden Konzentratbehälter werden beispielsweise für die Bicarbonatdialyse verwendet, wobei einer der Konzentratbehälter das Soll-Konzentrat und der andere Konzentratbehälter das Natriumbicarbonatkonzentrat enthält. Andererseits ist jedoch auch nur ein Konzentratanschluß ausreichend, sofern eine Dialysierflüssigkeit üblicher Zusammensetzung verwendet werden soll.

Vom dem Mischgerät 26 geht weiterhin eine weitere Zuleitung 40 ab, welche mit einer bilanziert zuführenden Wasserquelle 42 verbunden ist und in welcher eine weitere Pumpe 44 angeordnet ist.

In dem Mischgefäß 26 erfolgt die übliche Vermischung der Konzentrate mit Frischwasser mit einem Verhältnis von 1:34.

Stromab des Mischgefäßes 26 ist in die Zuleitung 22 eine Entgasungseinheit 46 eingeschaltet, mit der die frische Dialysierflüssigkeit von der darin enthaltenen Luft befreit wird. Diese Entgasungseinheit 46 besteht beispielsweise aus einer Drossel und einer stromab angeordneten Pumpe, mit der Unterdruck erzeugt wird. Die sie stromab der Pumpe ausscheidende Luft wird in das Mischgefäß 26 über eine nicht gezeigte Leitung zurückgeführt und dort ausgeschieden.

Stromab der Entgasungseinheit 46 ist weiterhin am Außenumfang der Zuleitung 22 eine Drossel 48 vorgesehen. Stromab der Drossel 48 ist in die Leitung 22 eine erste Leitfähigkeitszelle 50 eingeschaltet, mit welcher die Leitfähigkeitskonstante der Dialysierflüssigkeit überwacht bzw. bestimmt werden kann.

Stromab der Leitfähigkeitszelle 50 geht von der Zuleitung 22 eine Bypassleitung 52 zur Ableitung 24 ab. In der Bypassleitung 52 ist ein Bypassventil 54 vorgesehen.

Weiterhin zweigt stromab der Leitfähigkeitszelle 50 eine Leitung 56 ab, deren Ende mit einem Anschlußstück 58 versehen ist, welches mit dem nachstehend erläuterten arteriellen Ast bzw. dessen Anschlußstück verbindbar ist.

Stromab der Verzweigungspunkte der Bypassleitung 52 und der Leitung 56 ist in der Zuleitung 22 ein Dialysatorventil 60 vorgesehen, welchem in Richtung auf den Dialysator 12 ein Druckmessgerät folgt, welches ebenfalls mit der Leitung 22 verbunden ist.

Stromab des Dialysators 12 ist in der Ableitung 24 des Dialysierflüssigkeitswegs 20 eine zweite Leitfähigkeitszelle 64 vorgesehen, welche zur Feststellung der Leitfähigkeit der im Dialysator behandelten Dialysierflüssigkeit dienen kann. An diese Leitfähigkeitszelle 64 schließt sich ein weiteres Druckmeßgerät 66 an, das sich ebenso wie die Leitfähigkeitszelle noch stromauf der Abzweigstelle der Ableitung 24 mit der Bypassleitung 52 befindet.

In der Ableitung 24 ist weiterhin ein Blutleckdetektor 68 vorgesehen, an welchem sich stromab eine im Dialysierflüssigkeitsweg 20 eine Unterdruck erzeugende Pumpe 70 anschließt, welche stromab der Verzweigungstelle der Ableitung 24 mit der Bypassleitung 52 vorgesehen ist.

Nachfolgend zu der Pumpe 70 ist eine Testdrossel 72 vorgesehen, sowie eine Abzweigung in eine weitere Leitung 74, an deren Ende ebenfalls, wie die Leitung 56, ein Anschlußstück 76 vorgesehen ist. Dieses Anschlußstück 76 kann mit dem venösen Ast des Blutwegs verbunden werden.

Stromab der Leitung 74 zweigt eine weitere Leitung 78 ab, in welcher eine Pumpe 80 vorgesehen ist und deren Ende sich in die Leitung 82 und 84 verzweigt, in welchen Ventile 86 und 88 vorgesehen sind und deren Ende mit Konzentratbehältern 90 und 92 für Reinigungs- bzw. Desinfektionsmittelkonzentrate verbunden werden können.

Von der Leitung 82 oder 84 zweigt eine Abzweigleitung 85 ab, in welcher ein Ventil 87 vorgesehen ist und deren Ende vorteilhafterweise mit einem Hydrophobfilter 89 verschlossen ist. Durch diese Abzweigleitung 85 kann entweder Luft oder Wasser angesaugt werden, wodurch verhindert wird, daß die in den Konzentratbehältern 90 und 92 vorliegenden Reinigungs-bzw. Desinfektionsmittelkonzentrate eine chemische Reaktion eingehen können.

Weiterhin zweigt stromab der Leitung 78 eine Verbindungsleitung 94 von der Ableitung 24 ab, welche zum Mischgefäß 26 führt. In dieser Leitung 94 ist ein Ventil 96 vorgesehen. Nachfolgend zu dem Abzweigungspunkt der Leitung 94 ist der in Ableitung 24 ein Abflußventil 98 vorgesehen.

In dem Dialysierflüssigkeitsweg 20 bzw. in der Verbindungsleitung 94, insbesondere dem Mischgefäß 26, ist eine Einrichtung 100 zur Bestimmung des Dialysierflüssigkeitsniveaus angeordnet. Diese Einrichtung 100 ist mit einer Einrichtung 102 mittels einer elektrischen Leitung 104 verbunden, die weiterhin über eine elektrische Leitung 106 in Betriebsverbindung mit dem Ventil 96 steht. Zusätzlich ist eine Leitung 108 vorgesehen, um das Ventil 98 mit der Einrichtung 102 zu verbinden. Eine Leitung 110 stellt eine Verbindung mit der Pumpe 70 dar, während eine Leitung 112 die Einrichtung 102 mit der Druckmesseinrichtung 66 verbindet.

Das Hämodialysegerät 10 weist einen Blutweg 114 auf, welcher aus einer Zuleitung 116 besteht, in welcher eine Blutpumpe 118, beispielsweise in Form einer peristaltischen Pumpe angeordnet ist. Diese Zuleitung 116 ist mit dem Eingang der Kammer 18 des Dialysators 12 verbunden, während der Ausgang des Dialysators 12 in Verbindung mit einer Ableitung 120, in welche eine Tropfkammer 122 eingeschaltet ist.

Stromab der Tropfkammer 122 ist eine übliche Klemme 124 vorgesehen, welcher ein Luftsensor 126 folgt, der sowohl den Unterschied zwischen Luft und Blut als auch zwischen Kochsalzlösung und Luft detektieren kann.

Von dem Deckel der Tropfkammer 122 geht eine Leitung 128 ab, in welche ein venöses Druckmeßgerät 130 mündet. Von dieser Leitung 128 geht eine weitere Leitung 132 ab, die durch ein eingeschaltetes Luftventil 134 belüftet werden kann. Weiterhin ist die Leitung 132 über eine weitere Leitung 136 mit einer Luftförderpumpe 138 verbunden.

Das arterielle Leitungsstück, das heißt die Zuleitung 116 weist am Ende ein Anschlußstück 140 auf, das mit dem Anschlußsstück 58 verbunden werden kann, so wie es durch die gestrichelte Linie dargestellt ist. Weiterhin weist das venöse Ende des Blutwegs, das heißt die Ableitung 120 ein Anschlußstück 142 auf, welches, wie durch die gestrichelte Linie dargestellt, mit dem Anschlußstück 76 verbunden werden kann. Zusätzlich ist ein Kurzschlußstück 144 vorgesehen, das die Enden des Blutwegs, das heißt die beiden Anschlüsse 140 und 142 unter Bildung eines geschlossenen Kreislaufs verbinden kann.

Im folgenden wird die Betriebsweise des dargestellten Hämodialysegeräts 10 für die Wiederverwendung des Dialysators 12 beschreiben.

Nach Beendigung der Hämodialyse wird das im Blutweg 114 vorhandene Blut wie üblich unter Einsatz von Kochsalzlösung zu dem Patienten zurücktransportiert. Nach dem Abhängen des Hämodialysegeräts 10 von dem Patienten wird das arterielle Anschlußstück 140 mit dem Anschlußstück 58 des Dialysierflüssigkeitswegs 20 verbunden, während das venöse Ende über das Anschlußstück 142 mit dem venösen Anschlußstück 76 des Dialysierflüssigkeitswegs 20 verbunden wird. Demzufolge wird der Blutweg 114 mit dem Dialysierflüssigkeitsweg 20 verbunden, was dazu führt, daß das Blutschlauchsystem, das heißt die Leitungen 116 und 120 mit Dialysierflüssigkeit, Reinigungs- und/Desinfektionslösung behandelt werden kann.

Die gezeigte Vorrichtung weist weiterhin eine Leckprüfeinrichtung 150 auf, welche über elektrische Leitungen mit der Luftförderpumpe 138 (Leitung 154), dem Druckmeßgerät 66 (Leitung 158), der Pumpe 70 (Leitung 156), dem Druckmeßgerät 130 (Leitung 152) und dem Luftsensor 126 (Leitung 162) verbunden ist. Weiterhin kann das Luftventil 34 über eine Leitung 160 mit der Einrichtung 150 verbunden werden.

Zur Leckageprüfung wird erfindungsgemäß wie folgt vorgegangen:

Der mit Verschlußkappen versehene Dialysator wird zu Beginn des Prüfvorgangs am Hämodialysegerät angebracht, wobei der tieferliegende Dialysierflüssigkeitsanschluß mit der Dialysierflüssigkeitsablaufleitung 24 verbunden wird. Der am gegenüberliegenden Ende befindliche Blutanschluß wird mit dem venösen Schlauchsystem (Ableitung 120) verbunden. Nachfolgend wird die Verschlußkappe des obenliegenden Dialysierflüssigkeitsanschlusses geöffnet, so daß das in der Dialysierflüssigkeitskammer befindliche Desinfektionsmittel abfließen kann. Die Luftpumpe 138 pumpt nunmehr Luft in das venöse Schlauchsystem, welches einerseits am Dialysator 12 angeschlossen ist, andererseits durch die venöse Absperrklemme 124 verschlossen ist.

Durch diesen Vorgang wird die in der Blutkammer 18 befindliche Flüssigkeit aus dem Dialysator 12 in die Dialysierflüssigkeitskammer 16 verdrängt und fließt dort ab. Die Luft, welche sich in der Blutkammer 18 befindet, kann jedoch die mit Flüssigkeit gefüllte Membran 14 wegen der Kapilarkräfte nicht durchdringen. Dies macht sich in einem Druckanstieg in der Blutkammer 18 bemerkbar. Sobald dieser Druck einen Wert von etwa 500 mm/Hg erreicht hat, wird die Pumpe 138 abgeschaltet und es erfolgt eine Prüfung des Druckabfalls.

Erfindungsgemäß wird in dem zweiten Prüfungsschritt die Blutkammer 18 mit einem Unterdruck beaufschlagt oder evakuiert. Dies kann mit Hilfe einer Vakuumpumpe erfolgen, welche anstelle des Ventils 134 eingesetzt wird. Bei der gezeigten Anordnung ist die Pumpe 138 so ausgebildet, daß diese sowohl einen Unter- als auch einen Überdruck erzeugen kann.

Sobald die Luftpumpe 138 einen Unterdruck von etwa minus 500 mm/Hg erzeugt hat, wird die Pumpe angehalten und es erfolgt eine Überprüfung des Druckanstiegs in der Blutkammer 18.

Erfindungsgemäß wird ein Leck dann festgestellt, wenn die Prüfdrücke entweder nicht erreicht werden oder wenn der Druckabfall bzw. der Druckanstieg mehr als 20 mm/Hg pro Minute beträgt.

Nach erfolgter Prüfung wird das arterielle Schlauchsystem (Zuleitung 116) angeschlossen.

Es erfolgt nachfolgend eine Befüllung des Dialysators und des Blutschlauchsystems, wobei das Verfahren so ausgebildet sein kann, wie dies in der DE-PS 3418434 beschrieben ist.

Bei der erfindungsgemäßen Leckageprüfung ist das Hämodialysegerät 10 auf Durchfluß geschaltet, das heißt das Abflußventil 98 ist geöffnet, während das Rezirkulations ventil 96 geschlossen ist. Zugleich erfolgt die Wasserzuförderung über die Pumpe 44 und mit Hilfe des Druckmeßgeräts 66 wird die Unterdruckpumpe 70 durch die Leckprüfeinrichtung 150 auf einen bestimmten Transmembrandruck eingestellt. In dem Blutkreislauf, welcher kurzgeschlossen ist, wird die Blutpumpe 118 betätigt, während die Luftförderpumpe 138 in Betrieb genommen wird und solange Blut in das Blutschlauchsystem 114 pumpt, bis infolge des Druckunterschieds zwischen dem Blutkreislauf und dem Dialysierflüssigkeitsweg die im Blutkreislauf enthaltene Flüssigkeit über die Membran 14 in den Abfluß verdrängt wurde.

Nach dem Verdrängen der Flüssigkeit ist der Blutkreislauf mit Luft gefüllt, während auf der anderen Seite der Membran 14 in dem Dialysierflüssigkeitsweg Wasser vorhanden ist, welches mittels der Pumpe 70 entfernt wird. Die Luftförderpumpe 138 wird mit Hilfe der Leckprüfeinrichtung 150 auf einen Überdruck von etwa 500 mm/Hg eingestellt, bei welchem noch nicht die im Blutkreislauf enthaltene Luft durch die Poren der Membran 14 gedrückt wird. Der Druck im geschlossenen Blutkreislaufs wird mit Hilfe des venösen Druckmonitors 130 gemessen, wobei dieser Meßwert an die Leckprüfeinrichtung 150 abgegeben wird.

Sobald nach einer vorgegebenen Zeit die Flüssigkeit aus dem Blutkreislauf 114 durch die Luftförderpumpe 138 verdrängt worden ist, wird diese angehalten, wobei beim Weiterlaufen der Unterdruckpumpe 70 die am Druckmonitor 130 erhaltene Druckwert dahingehend beobachtet wird, ob der Druck abfällt oder konstant bleibt. Eine Änderung des Druckes dient als Indiz für ein Leck oder einen Defekt der Membran oder des zugehörigen Kreislaufs.

Die in dem zweiten Prüfungsschritt durchzuführende Prüfung erfolgt in analoger Weise.

Die Erfindung ist nicht auf das gezeigte Ausführungsbeispiel beschränkt, es ergeben sich vielmehr für den Fachmann vielfältige Abwandlungs- und Modifikationsmöglichkeiten.

## Patentansprüche

1. Verfahren zur Prüfung einer Hämodialysemembran eines Dialysators, welcher eine Blutkammer (18) und eine Dialysierflüssigkeitskammer (16) aufweist, bei welchem die in der Blutkammer (18) befindliche Flüssigkeit durch Luft ersetzt wird und in einem ersten Prüfschritt die Blutkammer (18) mit einem Überdruck beaufschlagt wird, dadurch gekennzeichnet, daß die Dialysierflüssigkeitskammer (16) vor dem ersten Prüfschritt mit Luft gefüllt wird und daß in einem zweiten Prüfschritt in der Blutkammer (18) ein Unterdruck erzeugt wird.

2. Verfahren nach Anspruch 1, dadurch gekennzeichnet, daß der Überdruck in der Blutkammer (18) etwa plus 500 mm/Hg entspricht.

3. Verfahren nach Anspruch 1 oder 2, dadurch gekenn zeichnet, daß der Unterdruck in der Blutkammer (18) etwa minus 500 mm/Hg beträgt.

4. Verfahren nach einem der Ansprüche 1 bis 3, dadurch gekennzeichnet, daß eine Leckanzeige betätigt wird, wenn der Überdruck und/oder der Unterdruck nicht erreicht wird.

5. Verfahren nach einem der Ansprüche 1 bis 4, dadurch gekennzeichnet, daß eine Leckanzeige betätigt wird, wenn der Druckanstieg und/oder der Druckabfall etwa 20 mm/Hg/min. beträgt.

6. Verfahren nach einem der Ansprüche 1 bis 5, dadurch gekennzeichnet, daß vor Beginn der Prüfung die Membran (14) mit Flüssigkeit gefüllt wird und daß vor dem ersten Prüfschritt zur Verdrängung der in der Blutkammer (18) befindlichen Flüssigkeit durch die Membran (14) in die Dialysierflüssigkeitskammer (16) die Blutkammer (18) mit Luft gefüllt wird und daß die Dialysierflüssigkeitskammer (16) zum Ablauf der Flüssigkeit mit einer Ablaufleitung verbunden wird.

## Claims

1. A process of testing the hemodialysis membrane of a dialyzer which comprises a blood chamber 18 and a dialysis fluid chamber (16) in which the fluid in the blood chamber (18) is substituted by air and in a first test step the blood chamber (18) is placed under over-pressure, characterized in that the dialysis fluid chamber (16) is filled with air before the first test step and in the second test step an under-pressure is generated in the blood chamber 18.

2. A process according to claim 1, characterized in that the over-pressure of the blood chamber (18) is approximately +500 mm/Hg.

3. A process according to claim 1, characterized in that the under-pressure in the blood chamber (18) is approximately -500 mm/Hg.

4. A process according to claim 1, characterized in that a leak indicator is activated when the over-pressure and/or the under-pressure are not reached.

5. A process according to claim 1, characterized in that a leak indicator is activated when the rate of pressure rise and or fall is of the order of 20 mm/Hg./minute.

6. A process according to claim 1, characterized in that before commencement of the test the membrane (14) is filled with fluid and before the first test step for the displacement of the fluid present in the blood chamber (18) through membrane (14) into dialysis chamber (16), the blood chamber (18) is filled with air and the dialysis chamber (16) is connected with a discharge means for the discharge of the fluid.

## Revendications

1. Procédé destiné à vérifier une membrane d'hémodialyse d'un dialyseur, qui comporte une chambre de sang (18) et une chambre de liquide de dialyse (16), dans lequel le liquide se trouvant dans la chambre de sang (18) est remplacé par de l'air et, dans une première étape d'essai, la chambre de sang (18) est soumise à une surpression, caractérisé en ce que la chambre de liquide de dialyse (16) est remplie avec de l'air, avant la première étape d'essai et en ce que dans une seconde étape d'essai, une dépression est produite dans la chambre de sang (18).

2. Procédé selon la revendication 1, caractérisé en ce que la surpression dans la chambre de sang (18) correspond à peu près à plus 500 mm/Hg.

3. Procédé selon la revendication 1 ou 2, caractérisé en ce que la dépression dans la chambre de sang (18) est égale à environ moins 500 mm/Hg.

4. Procédé selon l'une des revendications 1 à 3, caractérisé en ce qu'une indication de fuite est actionnée lorsque la surpression et/ou la dépression n'est pas atteinte.

5. Procédé selon l'une des revendications 1 à 4, caractérisé en ce qu'une indication de fuite est actionnée lorsque la montée de la pression et/ou la chute de la pression est environ égale à 20 mm/Hg/min.

6. Procédé selon l'une des revendications 1 à 5, caractérisé en ce qu'avant le début de la vérification, la membrane (14) est remplie de liquide et en ce qu'avant la première étape d'essai, la chambre de sang (18) est remplie d'air pour chasser le liquide se trouvant dans la chambre de sang (18), à travers la membrane (14), dans la chambre de liquide de dialyse (16) et en ce que la chambre de liquide de dialyse (16) est reliée à un conduite d'évacuation, pour évacuer le liquide.
